# EUROPÄISCHE PATENTANMELDUNG

(11) **EP 0 541 853 A1**
(43) Veröffentlichungstag der Anmeldung: **19.05.1993**
(21) Anmeldenummer: 91119520.4
(22) Anmeldetag: 15.11.1991
(51) Int. Cl.: B01D 11/02, A61K 35/78

(54) **Verfahren und Vorrichtung zur Konservierung von Extrakten aus Früchten der Sägezahnpalme (Serenoa repens)**

(71) Anmelder: MÜLLER-EXTRACT-COMPANY GmbH, D-96450 Coburg (DE)
(72) Erfinder: Müller, Adam, Dr.rer.nat., Rodacher Strasse 114 W-8630 Coburg (DE)
(74) Vertreter: KUHNEN, WACKER & PARTNER

(57) **Zusammenfassung**

Die Erfindung betrifft ein Verfahren und eine Vorrichtung zur Haltbarmachung von Extrakten (Primärextrakten), die aus Serenoa repens Früchten hergestellt wurden.

Die Konservierung wird durch die Behandlung der Primärextrakte in einer Hochdruck-Kolonne (C) mit unter-schiedlich temperierbaren Segmenten (C₁,C₂,C₃) durchgeführt.

Dabei findet eine fraktionierte Extraktion und Abscheidung in der Kolonne statt. Die unerwünschten Geruchs- und Geschmacksträger des Primärextraktes werden in einem außerhalb der Kolonne befindlichen Abscheidegefäß (S) abgetrennt.

Die nach der fraktionierten Extraktion in der Kolonne gewonnenen Sekundärextrakte sind wesentlich länger haltbar als die benutzten Startmaterialien.

## Beschreibung

Die Erfindung betrifft ein Verfahren und eine Vorrichtung zur Haltbarmachung von Extrakten hergestellt aus handelsüblichen Früchten der Sägezahnpalme (Serenoa repens).

Die Sägezahnpalme kommt in den Südstaaten der USA (Nord-und Süd-Carolina),Georgia und Florida vor. Die Früchte von Serenoa repens sind Beeren und bestehen aus der Fruchtschale, dem Fruchtfleisch, der Kernschale und dem Samenkern.

Pharmazeutisch wichtige Extrakte werden aus den getrockneten und zerkleinerten Beerenfrüchten durch Extraktion mit unpolaren Lösungsmitteln oder durch Extraktion mit überkritischer CO₂ hergestellt.

Die gewonnenen Extrakte sind Öle und werden als Primärextrakte bezeichnet. Sie enthalten unter anderem Fettsäuren der Reihen C₆ ₋ C₁₈, entsprechende Metyl- und Ethyl-Ester, gesättigte und ungesättigte Fettalkohole, Phytosterine, vor allem β-Sitosterin und Stigmasterin sowie andere Begleitstoffe.

Serenoa repens Extrakte sind therapeutisch wirksame Öle zur Behandlung des Prostata-Adenoms.

In der Französischen Patentschrift 2.480.754 wird ein Verfahren zur Herstellung von Serenoa Extrakten aus Früchten von Serenoa repens unter Verwendung von nicht polaren organischen Lösungsmitteln und dem Zusatz von Antioxidantien beschrieben.

Der Abbau von bestimmten, nicht verseifbaren Bestandteilen des Öles, wie z.B. die Phytosterine,insbesondere das β-Sitosterin werden hierdurch nicht gestoppt.

Das gleiche trifft auch für die aus Serenoa repens mit überkritischer CO₂ gewonnenen Öle entsprechend der Europäischen Patentanmeldung 0 250 953 zu.

Die durch CO₂ Extraktion aus Serenoa repens gewonnenen Öle haben den charakteristischen, unangenehm wirkenden Geruch und Geschmack wie z.B. die gelagerten Serenoa Früchte.Die gemäß der EPA 0250953 hergestellten Öle sind nicht über längere Zeit lagerfähig, sie werden insbesondere in ihrem Gehalt an β-Sitosterin fortlaufend abgebaut.

Das β-Sitosterin aus dem Öl der Serenoa repens Früchte ist genau so wichtig wie das reine β-Sitosterin, das ebenfalls zur Therapie des Prostata-Adenoms eingesetzt wird.

Aufgabe der vorliegenden Erfindung ist ein Verfahren und eine Vorrichtung, die den Abbau an Phytosterinen, insbesondere dem β-Sitosterin stoppt,die Haltbarkeit des behandelten Öles wesentlich verlängert und den unangenehm wirkenden Geruch und Geschmack beseitigt.

Bereits während der Lagerung der Serenoa Früchte entstehen diese unangenehmen Geruchs- und Geschmackstoffe, ausgelöst durch die Einwirkung von Hydrolasen im Fruchtfleisch mit dem Ergebnis, daß dieser charakteristische Geruch und Geschmack in den bisher hergestellten Extrakten wiedergefunden wurde.

Es war überraschend, daß nach dem in den Patentansprüchen beschriebenen Verfahren und der Vorrichtung durch eine fraktionierte Extraktion und Abscheidung in der Kolonne mit überkritischem Gas der Abbau der Phytosterine,besonders des β-Sitosterins gestoppt wird, die unangenehm wirkenden Geruchs- und Geschmacksträger des Startmaterials eliminiert und in dem außerhalb der Kolonne befindlichen Abscheidebehälter abgeschieden werden. Die Haltbarkeit des so behandelten und in der Kolonne gewonnenen Sekundärextraktes war wesentlich länger als erwartet.

Die Vorrichtung und das Verfahren der Erfindung sind so gestaltet, daß trotz der relativ leichten Löslichkeit des β-Sitosterins in überkritischer CO₂, dieses nur kurzfristig in bestimmten Abschnitten der Extraktionskolonne vom Öl abgetrennt ist, jedoch noch innerhalb der Kolonne an den Wänden in den Sumpfboden zurückfließt und dort mit dem gereinigten Öl zusammengeführt und abgezogen werden kann.

Die unangenehm wirkenden Geruchs- und Geschmackstoffe des Primärextraktes werden erfindungsgemäß zusammen mit einer öligen Matrix durch die überkritische CO₂ aus der Extraktionskolonne ausgetragen und im Abscheidebehälter von der mitgeführten CO₂ getrennt.

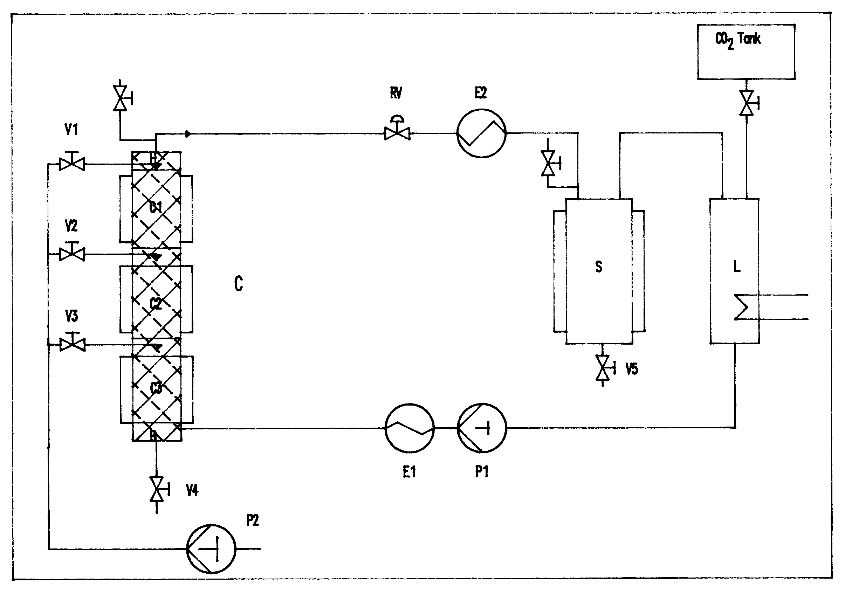

Im einzelnen wird das erfindungsgemäße Verfahren mit Hilfe der in der Zeichnung dargestellten Vorrichtung wie folgt durchgeführt:
Der für die Sekundärextraktion mit einem überkritischen Gas, wie z.B. CO₂ erforderliche Druck und auch die notwendige Durchflußmenge werden durch die Druckerhöhungspumpe P₁ geregelt. Der Druck liegt zwischen 75 und 350 bar. Im Wärmetauscher E₁ findet eine Temperierung der CO₂ auf eine Mindesttemperatur von +20^{o} C statt. Die tatsächlich notwendige Extraktionstemperatur wird in den als Doppelmantel ausgebildeten Wärmetauschern der Kolonne und zwar in den Kolonnenabschnitten C₁,C₂ und C₃ geregelt. Dort können Extrationstemperaturen von + 20^{o} C bis + 90^{o} C erzeugt werden. Jeder Kolonnenabschnitt ist mit einem unabhängig vom anderen Abschnitt arbeitenden Temperierungssystem ausgestattet, sodaß erfindungsgemäß mit Temperaturgradienten gefahren werden kann. Die einzelnen Kolonnenabschnitte sind ferner mit Packungen zur besseren Verteilung und Förderung der Löslichkeit des eingespritzten Primärextraktes gefüllt.

Je nach den gewählten Verfahrensbedingungen wird der Primärextrakt in Höhe der Kolonnenabschnitte C₃, C₂ oder C₁ über die Ventile V₃, V₂ oder V₁ in die Querschnittsmitte der Kolonne eingespritzt Das als Lösungsmittel benutzte unterkritische/überkritische Gas strömt vom Sumpfboden B durch die einzelnen Kolonnenabschnitte zum Kopf der Kolonne H. Wesentlich für die Trennung in der überkritischen Gasphase ist, daß die eingespritzten Primärextrakte zuerst als Flüssigkeit in die Mitte des Querschnittes der Kolonne gelangen, wo sie aufgrund der Beschaffenheit der Packungen, z.B. Sulzerpakkungen, durch das Lösungsmittel extrahiert werden. Soweit unmittelbar nach dem Lösevorgang wieder eine Abscheidung erfolgt, findet diese peripher an der Wand eines Kolonnensegmentes statt. Der abgetrennte Extrakt fließt an der Kolonnenwand von oben nach unten und wird im Sumpfboden S als gereinigter und lagerstabiler Sekundärextrakt gewonnen.Durch das ständige Wechselspiel zwischen Lösung und Abscheidung mit Hilfe der Packungen und der unterschiedlichen Temperaturbedingungen in den einzelnen Kolonnenabschnitten wird erfindungsgemäß eine fraktionierte Extraktion in der Kolonne durchgeführt. Durch diese Verfahrensweise ist es erfindungsgemäß möglich, auch relativ leicht in überkritischer CO₂ lösliche Komponenten, wie z.B. das β-Sitosterin noch in der Extraktionskolonne abzuscheiden und direkt dem gereinigten Sekundärextrakt im Sumpfboden zu zuführen.

Andere Komponenten wie z.B. die unerwünschten Geruchs- und Geschmackstoffe werden mit einer ölähnlichen Matrix und der überkritischen CO₂ über das Regelventil R_{V} und den Wärmetauscher E₂ in den Abscheider S überführt, wo sie von der CO₂ abgetrennt werden. Durch das Ventil V₅ kann der erhaltene Extrakt kontinuierlich ausgetragen werden. Die Extraktausbeute bestehend aus öliger Matrix, die stark stechend und äußerst unangenehm riecht, geschmacklich wirkte diese Flüssigkeit ätzend, schwankt zwischen 2% und 12% bezogen auf den eingesetzten Primärextrakt.

Die abgetrennte CO₂ wird in den Verflüssiger L als Flüssigkeit überführt und steht dort wieder der Druckerhöhungspumpe P₁ zur Verfügung.

Wichtig zur Durchführung des erfindungsgemäßen Verfahrens ist, daß die Kolonne mindestens 2 Kolonnenabschnitte mit unabhängig voneinander arbeitenden Temperaturzonen besitzt. Bevorzugt werden 3 oder mehr Temperaturzonen, die unabhängig voneinander temperierbar sind.

In dem untersten Kolonnenabschnitt (C₃), der direkt über der Sumpfzone liegt, kann die Temperatur des Lösungsmittels unterkritisch, nahe dem kritischen Punkt oder überkritisch sein.Das gleiche gilt auch für den Kolonnenabschnit C₂, der Kolonnenabschnitt C₁ muß immer mit überkritischer Temperatur des Lösungsmittels arbeiten, der Druck des Lösungsmittels ist in allen Kolonnenabschnitten überkritisch.

Die nun folgenden Beispiele erläutern die Erfindung:

### Beispiel 1, Extraktionsversuch:

Es wurden 500 ml Serenoa Repens Primärextrakt nach dem Verfahren der EPA 0250 953 Beispiel 1 und 500 ml Primärextrakt entsprechend der EPA 0250 953 Beispiel 2 hergestellt und miteinander vermischt.

Diese Mischung war das Startmaterial für die Sekundärextraktion an der Hochdruckkolonne.

In die Kolonne, bestehend aus drei unabhängig voneinander temperierbaren Segmenten, die mit Sulzerpackungen gefüllt waren, wurden 1000 ml Primärextrakt während einer Stunde kontinuierlich über Ventil V₂ in Höhe zwischen Segment C₁ und C₂ in die Querschnittsmitte der Kolonne eingespritzt. Der Extraktionsdruck betrug am Injektionspunkt (Querschnittsmitte der Kolonne) 150 bar, an der Kolonnen-wand 90 bar.

Die Extraktionstemperatur im Kolonnensegment C₁ war 90^{o} C, im Kolonnensegment C₂ 60^{o} C und im Kolonnensegment C₃ 28^{o} C. Am Einspritzpunkt des Primärextraktes wurden 80⁰C gemessen.

Als Extraktionsmittel wurde überkritische CO₂ benutzt, die vom Sumpfboden durch die Packungen der einzelnen Segmente strömte und schließlich am Kopf der Kolonne mit den noch in CO₂ gelösten Stoffen im Abscheidebehälter S bei 50 bar und 25^{o} C abgeschieden wurde.

Im Abscheidebehälter S wurden 50 ml einer bräunlichen, öligen Flüssigkeit von äußerst stechendem Geruch und ätzendem Geschmack (Sek. 2) abgeschieden.

Aus dem Sumpfboden B wurden über das Ventil V₄ 940 ml eines gereinigten, klaren, gelblichen, nahezu geruchs- und geschmacksneutralen Öles (Sek. 1) gewonnen.

| | |
|---|---|
| Dementsprechend war die Ausbeute an Sekundärextrakt 1 (Sek. 1) in der Kolonne | 94 %. |
| Die Ausbeute an Sekundärextrakt 2 (Sek. 2) im Abscheidebehälter war | 5 %. |

| | Prim.Extr. | Sek.1 | Sek.2 |
|---|---|---|---|
| Dichte bei 15^{o} C | 0,925 | 0,915 | 0,945 |
| Brechungsindex bei 20^{o} C | 1,45 | 1,47 | 1,41 |
| nichtverseifbare Anteile | 2,75% | 2,86% | 0,15% |
| Gehalt an freien Fettsäuren | 87% | 84% | 92% |
| β-Sitosterin (LC/GC) | 1,45 % | 1,35% | Spuren |

### Beispiel 2, Lagerungsversuche:

4 x 50 ml einer Mischung des für das Erfindungsbeispiel 1 benutzten Startmaterials (Primärextrakt)wurden in Glasflaschen gefüllt, verschlossen und im Forciertest im Wärmeschrank 8 Wochen lang bei 45^{o}C gelagert.

In gleicher Weise wurden 4 x 50 ml des nach dem Erfindungsbeispiel 1 gewonnen Sekundärextraktes (Sek. 1) bei 45⁰C 8 Wochen lang gelagert.

Als Indikator der Lagerstabilität wurde der Gehalt an β-Sitosterin durch gekoppelte LC/GC ermittelt.

| | | β-Sitosterin % |
|---|---|---|
| Primär Extrakt | vor | 1,45 |
| Primär Extrakt | nach | 0,22 |
| Sek 1 | vor | 1,35 |
| Sek 1 | nach | 1,25 |
| vor - Analyse vor der Lagerung, nach - nach der Lagerung. | | |

### Organoleptische Beurteilung:

Der Primärextrakt vor der Lagerung zeigte eine orangegelbe Farbe, nach der Lagerung war er braun. Die Prüfung der Qualität auf Geruchs- und Geschmackstoffe ergab, daß die schon vor der Lagerung festgestellten unangenehmen Eigenschaften nach der Lagerung in noch stärkerem Maße feststellbar waren.

Der Sekundärextrakt (Sek 1) hatte vor der Lagerung eine gelbe Farbe, durch die Lagerung hatte sich die Farbe nicht verändert.Geruchlich und geschmacklich waren keine signifikanten Unterschiede gegenüber dem Sek 1 vor der Lagerung festzustellen.

Der Umfang der Erfindung ist nicht begrenzt auf die Bedingungen der Beispiele.

## Patentansprüche

1. Verfahren zur Haltbarmachung von Extrakten aus Früchten der Sägezahnpalme (Serenoa repens) dadurch gekennzeichnet, daß der zu behandelnde Extrakt in unterschiedlich temperierten Zonen einer Kolonne mit einem überkritischen Gas einer fraktionierten Extraktion und fraktionierten Abscheidung in der Kolonne unterworfen wird und die unerwünschten Geruchs- und Geschmackstoffe in einem Abscheidebehälter außerhalb der Kolonne abgetrennt werden.

2. Verfahren nach Anspruch 1 dadurch gekennzeichnet, daß die fraktionierte Extraktion und Abscheidung in den unterschiedlich temperierten Zonen der Kolonne stattfindet

3. Verfahren nach Anspruch 2 dadurch gekennzeichnet, daß in den unterschiedlich temperierten Zonen eine Abscheidung der im überkritischen Gas leichtlöslichen Substanzen stattfindet.

4. Verfahren nach Anspruch 1 - 2 dadurch gekennzeichnet, daß die Abscheidung der unerwünschten Geruch- und Geschmackstoffe in einem Abscheidebehälter außerhalb der Kolonne bei Temperaturen von +15 bis +30^{o}C und Drücken von 40 - 75 bar erfolgt.

5. Verfahren nach Anspruch 1 - 3 dadurch gekennzeichnet,daß zur fraktionierten Extraktion und Abscheidung in der Kolonne Drücke von 75 - 350 bar und Temperaturen von +20^{o}C bis +90^{o}C angewendet werden.

6. Verfahren nach Anspruch 1 - 5 dadurch gekennzeichnet, daß als überkritisches Gas CO₂ verwendet wird.

7. Vorrichtung zur Durchführung des Verfahrens nach Anspruch 1 - 6 dadurch gekennzeichnet, daß eine Hochdruckextraktionsanlage mit Kolonne gemäß Zeichnung benutzt wird.

8. Vorrichtung zur Durchführung des Verfahrens nach Anspruch 1 - 6 dadurch gekennzeichnet, daß eine Kolonne mit verschiedenen Segmenten (C₁ - C₃ der Zeichnung) und einem Abscheidegefäß S benutzt wird.

9. Vorrichtung zur Durchführung des Verfahrens nach Anspruch 1 - 6 dadurch gekennzeichnet, daß die verschiedenen Segmente der Kolonne unabhängig voneinander temperierbar sind.

10. Vorrichtung zur Durchführung des Verfahrens nach Anspruch 1 - 6 dadurch gekennzeichnet, daß die Kolonne mit Pakkungen ausgestattet ist.
